# EUROPEAN PATENT APPLICATION

(11) **EP 4 306 983 A1**
(43) Date of publication of application: **17.01.2024**
(21) Application number: 22184067.1
(22) Date of filing: 11.07.2022
(51) Int. Cl.: G01R 33/56, G01R 33/54, G01R 33/48, A61B 5/055, G16H 30/20, G06T 7/00, A61B 5/00, G06N 3/08

(54) **MAKING ANATOMICAL MEASUREMENTS USING MAGNETIC RESONANCE IMAGING**

(71) Applicant: Koninklijke Philips N.V., 5656 AG Eindhoven (NL)
(72) Inventor: PEZZOTTI, Nicola, Eindhoven (NL); WENZEL, Fabian, Eindhoven (NL); VAN DEN BRINK, Johan Samuel, 5656AG Eindhoven (NL); WEESE, Rolf Jürgen, 5656AG Eindhoven (NL); FLAESCHNER, Nick, Eindhoven (NL); DONEVA, Mariya Ivanova, Eindhoven (NL); EWALD, Arne, Eindhoven (NL)
(74) Representative: Philips Intellectual Property & Standards

(57) **Abstract**

Disclosed herein is a medical system (100, 300, 500). The execution of machine executable instructions (112) causes a computational system (104) to: receive (200) a baseline anatomical measurement (114) descriptive of a clinical magnetic resonance image of a subject (318); receive (202) scan metadata (116) descriptive of the clinical magnetic resonance image of the subject; send (204) scan parameters via a network connection (350) to a low-field magnetic resonance imaging system (301); receive (206) subsequent k-space data (122) from the low-field magnetic resonance imaging system via the network connection in response to sending the scan parameters; reconstruct (208) a subsequent magnetic resonance image (124) from the subsequent k-space data; determine (210) a subsequent anatomical measurement (128) in response to inputting the subsequent magnetic resonance image into the segmentation module; and provide (212) a warning signal (132) if the subsequent anatomical measurement varies from the baseline anatomical measurement by more than a predetermined amount.

## Description

### FIELD OF THE INVENTION

The invention relates to magnetic resonance imaging, in particular to making anatomical measurements.

### BACKGROUND OF THE INVENTION

A large static magnetic field is used by Magnetic Resonance Imaging (MRI) scanners to align the nuclear spins of atoms as part of the procedure for producing images within the body of a patient. A main magnetic is used to generate a large static magnetic field is referred to as the B0 field or the main magnetic field. Various quantities or properties of the subject can be measured spatially and imaged using MRI.

One difficulty with MRI is that the equipment is expensive. Installing an operating a MRI scanner may be a major expense for a hospital. It is possible to save costs by using a main magnet that generates a lower B0 field. However, this may have the effect of reducing the resolution and/or the signal to noise ratio for MRI images. Typically, these low-field MRI images are of such poor quality that they are not desirable.

The journal article Wu Z, Chen W, Nayak KS. Minimum Field Strength Simulator for Proton Density Weighted MRI. PLoS One. 2016;11(5) e0154711. doi:10.1371/journal.pone.0154711. PMID: 27136334; PMCID: PMC4852924 discloses a framework for simulating low-field proton-density weighted MRI acquisitions based on high-field acquisitions.

### SUMMARY OF THE INVENTION

The invention provides for a medical system, a computer program product, and a method in the independent claims. Embodiments are given in the dependent claims.

Embodiments may provide for a means of performing follow-up of subsequent magnetic resonance imaging scans using so called low-field magnetic resonance imaging systems. To do this a baseline anatomical measurement obtained from a clinical magnetic resonance image and scan metadata descriptive of the baseline anatomical measurement are provided. The scan metadata and the baseline anatomical measurement are used to provide scan parameters which are sent via a network connection to a low-field magnetic resonance imaging system. Subsequent k-space data is received from the low-field magnetic resonance imaging system in response to the scan parameters and this is used to reconstruct a subsequent magnetic resonance image. The scan metadata describe the clinical magnetic resonance image as having a first resolution and a first signal to noise ratio. The subsequent magnetic resonance image has a second resolution and a second signal to noise ratio. The first signal to noise ratio is higher than the second signal to noise ratio and/or the first resolution is higher than the second resolution. And a subsequent anatomical measurement. A subsequent anatomical measurement is then determined by inputting the subsequent magnetic resonance image into a segmentation module. The subsequent anatomical measurement may then be compared to the baseline anatomical measurement. Although the low-field magnetic resonance imaging system may not have a sufficiently strong main magnetic field to produce a clinical quality image, it may still be used to provide anatomical information.

In one aspect the invention provides for a medical system that comprises a memory storing machine-executable instructions. The medical system further comprises a computational system. Execution of the machine-executable instructions causes the computational system to receive a baseline anatomical measurement descriptive of a clinical magnetic resonance image of a subject. Execution of the machine-executable instructions further causes the computational system to receive scan metadata descriptive of the clinical magnetic resonance image of the subject. The scan metadata comprises scan coordinates referenced to predetermined anatomical landmarks of the subject. The scan metadata is descriptive of the clinical magnetic resonance image having a first resolution and a first signal-to-noise ratio.

The baseline anatomical measurement may take different forms in different examples. For example, the baseline anatomical measurement could be a distance that is referenced relative to the predetermined anatomical landmarks. In another example, the baseline anatomical measurement could be a size, volume, or other physical quantity descriptive of an anatomical structure such as an organ and/or tumor. In some examples the baseline anatomical measurement may be a numerical value. In other examples, the baseline anatomical measurement may be a line or segmentation within the clinical magnetic resonance image. In examples it is not necessary to actually have the clinical magnetic resonance image there because the baseline anatomical measurement is provided as well as the scan metadata which provides such things as the scan coordinates, the first resolution, and the first signal-to-noise ratio.

Execution of the machine-executable instructions further causes the computational system to send scan parameters via a network connection to a low-field magnetic resonance imaging system. A low-field magnetic resonance imaging system as used herein encompasses a magnetic resonance imaging system with a magnetic field that is lower than is typically used for clinical magnetic resonance imaging. For example, the low-field magnetic resonance imaging system could for example have a main magnetic field strength less than 0.6 or even 0.2 T. The scan parameters may be provided from several different sources; they could of course be standard parameters used by the low-field magnetic resonance imaging system or they could be scan parameters which are optimized for measuring the particular baseline anatomical measurement in subsequent images.

Execution of the machine-executable instructions further causes the computational system to receive subsequent k-space data from the low-field magnetic resonance imaging system via the network connection in response to sending the scan parameters. The subsequent k-space data was acquired using the scan parameters that were sent to the low-field magnetic resonance imaging system. Execution of the machine-executable instructions further causes the computational system to reconstruct a subsequent magnetic resonance image from the subsequent k-space data. Execution of the machine-executable instructions further causes the computational system to determine a subsequent anatomical measurement in response to inputting the subsequent magnetic resonance imaging data into the segmentation module.

The segmentation module may perform several different tasks. It may for example be used to perform a segmentation or measurement in the subsequent magnetic resonance image. The subsequent magnetic resonance image has a second resolution that may be lower than the first resolution and/or have a signal-to-noise ratio that is lower than the first signal-to-noise ratio. This may be the consequence of using a low-field magnetic resonance image. Execution of the machine-executable instructions further causes the computational system to provide a warning signal if the subsequent anatomical measurement varies from the baseline anatomical measurement more than the predetermined amount. For example, if the baseline anatomical measurement is a distance, if the distance changes more than a certain amount then this may trigger the warning signal. If the anatomical measurement being compared is for example a tumor size or other volume measurement, an increase in size or volume of the object being measured may also be used to trigger the warning signal. This embodiment may be beneficial because it may provide for a means of more inexpensively monitoring an anatomical measurement of a subject. For example, when doctors perform a diagnosis or examination of a subject, they may use a clinical magnetic resonance imaging system that may for example have a field strength greater than 0.6 or greater than even 1 T. These systems may be more expensive than low-field magnetic resonance imaging systems. The medical system may for example be located in one example, as a cloud service or remote server that may provide image reconstruction and other services to a variety of low-field magnetic resonance imaging systems.

The warning signal may take different forms in different examples. In one example, it may be a signal that is provided to a physician via an email or even via a cellular telecommunication system. In other examples, the warning signal could be a message or instructions that are then sent to the low-field magnetic resonance imaging system so that they are received by the subject. This for example, could be instructions to perform various health tasks such as instructions for a healthcare provider or for specific changes in behavior for the subject such as adjusting food consumption or taking other actions. In other examples the warning signal could be used to send further scan parameters to the low-field magnetic resonance imaging system to repeat the acquisition of the subsequent k-space data. For example, if the anatomical measurement indicated that there may be a problem or health issue with the subject, the providing of the warning signal could be used to automatically retake the measurement to confirm that the subsequent anatomical measurement is correct.

In another aspect the medical system further comprises the low-resolution magnetic resonance imaging system. The low-resolution magnetic resonance imaging system comprises a local memory and a controller. The local memory stores survey scan pulse sequence commands and measurement pulse sequence commands. The local memory further stores controller commands. Execution of the controller commands causes the controller to receive the scan parameters via the network connection. Execution of the controller commands further causes the controller to acquire survey scan k-space data by controlling a low-resolution magnetic resonance imaging system with the survey scan pulse sequence commands. Execution of the controller commands further causes the controller to reconstruct a survey scan image from the survey scan k-space data. Execution of the controller commands further causes the controller to detect a location of the predetermined anatomical landmark of the subject in the survey scan image.

For example, a segmentation module that identifies the predetermined anatomical landmarks of the subject may be used to provide this location. Other things such as segmentation module, such as a deformable shape model may also be used for determining the location of the predetermined anatomical landmarks. Execution of the controller commands further causes the controller to adjust the acquisition pulse sequence commands using the location of the predetermined anatomical landmarks of the subject in the survey scan image, the second resolution, and the scan coordinates referenced to predetermined anatomical landmarks of the subject. Execution of the controller commands further causes the controller to acquire the subsequent k-space data by controlling the low-resolution magnetic resonance imaging system with the acquisition pulse sequence commands. Execution of the controller commands further causes the controller to send the subsequent k-space data to the computational system via the network connection. This embodiment may be beneficial because it may provide for a means of remotely acquiring magnetic resonance images automatically.

In another embodiment the low-resolution magnetic resonance imaging system comprises a main magnet configured for generating a main magnetic field. The main magnetic field has a strength of 0.6 T or less. In another embodiment the main magnetic field preferably has a strength of 0.2 T or less. The use of a main magnet with a field strength of 0.6 T may have the advantage that it is much less expensive to build and operate. As such, a magnet with a field strength of 0.2 T or less has an even lower cost to build and maintain. The disadvantage of using a magnet with such a low field strength is that the signal-to-noise may be lower as well as it may be more difficult to measure with the same resolution.

In another embodiment the controller is configured to perform the acquisition of the subsequent k-space data and sending of the subsequent k-space data to the computational system automatically upon receipt of the scan parameters. This embodiment may be advantageous because it may eliminate the need for a trained operator of the low-field magnetic resonance imaging system.

In another embodiment the memory further stores a scan parameter configuration module. The scan parameter configuration module is configured to output the scan parameters in response to receiving the second resolution and/or the second signal-to-noise ratio, the scan coordinates, and the baseline anatomical measurement. Execution of the machine-executable instructions further causes the computational system to receive the scan parameters in response to inputting the second resolution and/or the second signal-to-noise ratio, the scan coordinates, and the baseline anatomical measurement into the scan parameter configuration module. This embodiment may be beneficial because the scan parameter configuration module can be used to automatically generate the scan parameters which enable the low-field magnetic resonance imaging system to accurately measure the anatomical measurement although it has a lower field and probably a lower quality than the clinical magnetic resonance imaging system used to obtain the baseline anatomical measurement.

In another embodiment the scan parameter configuration module is at least partially implemented as a lookup table, a neural network, or an expert system. Over time, as the system is used for a larger and larger number of examinations, the data about what the proper scan parameters were for a particular configuration and baseline anatomical measurement can be stored and then used later. In other examples a neural network can be trained for this. Likewise, the data acquired over repeated use can be encoded in an expert system which can be used to automatically provide the scan parameters.

In another embodiment the scan parameters comprise a selection of a pulse sequence type. The baseline anatomical measurement could be derived from the clinical magnetic resonance image having a particular weighting or image type. This image type for example could be stored in the scan metadata and this could then be used to select a pulse sequence type for acquiring the subsequent k-space data on the low-field magnetic resonance imaging system.

In another embodiment the computational system is implemented as a cloud computing system. This may be beneficial because it may enable the computational system to serve a large number of low-field magnetic resonance imaging systems. For example, if the 1 low-field magnetic resonance imaging systems were located in smaller clinics or stores in a distributed fashion. The use of a cloud computing system enables a single or very few medical systems to provide the reconstruction and determination of the subsequent anatomical measurement.

In another embodiment the segmentation module is implemented as a neural network. The neural network may for example be a U-net or a ResNet. The training of the neural network may be special in several embodiments. The baseline anatomical measurement is taken from the clinical magnetic resonance image which has a first resolution and a first signal-to-noise ratio. As the subsequent magnetic resonance image is used to determine the subsequent anatomical measurement the data comes from a magnetic resonance imaging system with likely a lower magnetic field and/or image quality. To properly train the neural network one could take a training image and then either automatically or manually segment this image to provide the baseline anatomical measurement. This training anatomical measurement may then be used as part of the training data. The training magnetic resonance image could then be resampled and then the lower resolution and lower signal-to-noise could be simulated to produce an image which can then be input into the neural network during training. The lower resolution image can be input into the neural network and then the segmentation or anatomical measurement derived from the original image can be used as the ground truth data. In this way, the neural network can be trained to produce an anatomical measurement as if the measurement were performed on a higher resolution image.

In another embodiment the neural network is trained by repeatedly receiving a training clinical magnetic resonance image or training clinical k-space data. The training clinical magnetic resonance image has the first resolution and/or the first signal-to-noise ratio. The neural network is further trained by repeatedly receiving a training anatomical measurement descriptive of the baseline anatomical measurement on the training clinical magnetic resonance image. As was previously mentioned, this could for example be performed manually or be performed by a segmentation module. The neural network is further trained by repeatedly calculating a simulated subsequent magnetic resonance image with the second resolution and/or the second signal-to-noise ratio. This may be either the training clinical magnetic resonance image or the training clinical k-space data. The neural network is further trained by repeatedly constructing training data from pairs of the simulated subsequent magnetic resonance image and the training anatomical measurement. The neural network is further trained by repeatedly training the neural network using the training data. This for example may be performed using a deep learning algorithm.

In another embodiment the medical system further comprises a clinical magnetic resonance imaging system. The clinical magnetic resonance imaging system is configured to acquire the clinical magnetic resonance image of the subject and determine the baseline anatomical measurement from the clinical magnetic resonance image. The baseline anatomical measurement may either be provided automatically by an algorithm or a neural network or it may be provided by data which was received by a physician or operator of the clinical magnetic resonance imaging system. The clinical magnetic resonance imaging system is further configured to provide the baseline anatomical measurement to the computational system. It for example could provide this data via a network or other connection.

In another aspect the invention provides for a method of medical imaging. The method comprises receiving a baseline anatomical measurement descriptive of a clinical magnetic resonance image of the subject. The method further comprises receiving scan metadata descriptive of the clinical magnetic resonance image of the subject. The scan metadata comprises scan coordinates referenced to predetermined anatomical landmarks of the subject. The scan metadata is descriptive of the clinical magnetic resonance image having a first resolution and/or a first signal-to-noise ratio. The method further comprises sending scan parameters via a network connection to a low-field magnetic resonance imaging system via a network connection. The scan parameters comprise a second resolution and/or a second signal-to-noise ratio. The scan parameters further comprise the scan coordinates referenced to the predetermined anatomical landmarks of the subject. The first resolution is higher than the second resolution. The first signal-to-noise ratio is higher than the second signal-to-noise ratio. The method further comprises receiving subsequent k-space data from the low-field magnetic resonance imaging system via the network connection in response to sending the scan parameters.

The method further comprises reconstructing a subsequent magnetic resonance image from the subsequent k-space data. The method further comprises determining a subsequent anatomical measurement in response to inputting the subsequent magnetic resonance image into the segmentation module. The method further comprises providing a warning signal if the subsequent anatomical measurement varies from the baseline anatomical measurement by more than a predetermined amount.

In another embodiment the medical system further comprises the low-resolution magnetic resonance imaging system. The method further comprises receiving the scan parameters via the network connection. The method further comprises acquiring survey scan k-space data by controlling the low-resolution magnetic resonance imaging system with the survey scan pulse sequence commands. The method further comprises reconstructing a survey scan image from the survey scan k-space data. The method further comprises detecting a location of the predetermined anatomical landmarks of the subject in the survey scan image. This may for example be done using a segmentation algorithm. The method further comprises adjusting the acquisition pulse sequence commands using the location of the predetermined anatomical landmarks of the subject in the survey scan image, the second resolution, and the scan coordinates referenced to the predetermined anatomical landmarks of the subject. The method further comprises acquiring the subsequent k-space data by controlling the low-resolution magnetic resonance imaging system with the acquisition pulse sequence commands. The method further comprises sending the subsequent k-space data to the computational system via the network connection.

In another aspect the invention provides for a computer program product comprising machine-executable instructions for execution by a computational system controlling the medical system. Execution of the machine-executable instructions causes the computational system to receive a baseline anatomical measurement descriptive of a clinical magnetic resonance image of the subject. Execution of the machine-executable instructions further causes the computational system to receive scan metadata descriptive of the clinical magnetic resonance image of the subject. The scan metadata comprises scan coordinates referenced to predetermined anatomical landmarks of the subject. The scan metadata is descriptive of the clinical magnetic resonance image having a first resolution and/or a first signal-to-noise ratio.

Execution of the machine-executable instructions further causes the computational system to send scan parameters via a network connection to a low-field magnetic resonance imaging system via a network connection. The scan parameters comprise a second resolution and/or a second signal-to-noise ratio. The scan parameters further comprise scan coordinates referenced to the predetermined anatomical landmarks of the subject. The first resolution is higher than the second resolution and/or the first signal-to-noise ratio is higher than the second signal-to-noise ratio. Execution of the machine-executable instructions further causes the computational system to receive subsequent k-space data from the low-field magnetic resonance imaging system via the network connection in response to sending the scan parameters. Execution of the machine-executable instructions further causes the computational system to reconstruct a subsequent magnetic resonance image from the subsequent k-space data. Execution of the machine-executable instructions further causes the computational system to determine a subsequent anatomical measurement in response to inputting the subsequent magnetic resonance image into the segmentation module. Execution of the machine-executable instructions further causes the computational system to provide a warning signal if the subsequent anatomical measurement varies from the baseline anatomical measurement by more than a predetermined amount.

It is understood that one or more of the aforementioned embodiments of the invention may be combined as long as the combined embodiments are not mutually exclusive.

As will be appreciated by one skilled in the art, aspects of the present invention may be embodied as an apparatus, method or computer program product. Accordingly, aspects of the present invention may take the form of an entirely hardware embodiment, an entirely software embodiment (including firmware, resident software, micro-code, etc.) or an embodiment combining software and hardware aspects that may all generally be referred to herein as a "circuit," "module" or "system." Furthermore, aspects of the present invention may take the form of a computer program product embodied in one or more computer readable medium(s) having computer executable code embodied thereon.

Any combination of one or more computer readable medium(s) may be utilized. The computer readable medium may be a computer readable signal medium or a computer readable storage medium. A 'computer-readable storage medium' as used herein encompasses any tangible storage medium which may store instructions which are executable by a processor or computational system of a computing device. The computer-readable storage medium may be referred to as a computer-readable non-transitory storage medium. The computer-readable storage medium may also be referred to as a tangible computer readable medium. In some embodiments, a computer-readable storage medium may also be able to store data which is able to be accessed by the computational system of the computing device. Examples of computer-readable storage media include, but are not limited to: a floppy disk, a magnetic hard disk drive, a solid state hard disk, flash memory, a USB thumb drive, Random Access Memory (RAM), Read Only Memory (ROM), an optical disk, a magneto-optical disk, and the register file of the computational system. Examples of optical disks include Compact Disks (CD) and Digital Versatile Disks (DVD), for example CD-ROM, CD-RW, CD-R, DVD-ROM, DVD-RW, or DVD-R disks. The term computer readable-storage medium also refers to various types of recording media capable of being accessed by the computer device via a network or communication link. For example, data may be retrieved over a modem, over the internet, or over a local area network. Computer executable code embodied on a computer readable medium may be transmitted using any appropriate medium, including but not limited to wireless, wire line, optical fiber cable, RF, etc., or any suitable combination of the foregoing.

A computer readable signal medium may include a propagated data signal with computer executable code embodied therein, for example, in baseband or as part of a carrier wave. Such a propagated signal may take any of a variety of forms, including, but not limited to, electro-magnetic, optical, or any suitable combination thereof. A computer readable signal medium may be any computer readable medium that is not a computer readable storage medium and that can communicate, propagate, or transport a program for use by or in connection with an instruction execution system, apparatus, or device.

'Computer memory' or 'memory' is an example of a computer-readable storage medium. Computer memory is any memory which is directly accessible to a computational system. 'Computer storage' or 'storage' is a further example of a computer-readable storage medium. Computer storage is any non-volatile computer-readable storage medium. In some embodiments computer storage may also be computer memory or vice versa.

A 'computational system' as used herein encompasses an electronic component which is able to execute a program or machine executable instruction or computer executable code. References to the computational system comprising the example of "a computational system" should be interpreted as possibly containing more than one computational system or processing core. The computational system may for instance be a multi-core processor. A computational system may also refer to a collection of computational systems within a single computer system or distributed amongst multiple computer systems. The term computational system should also be interpreted to possibly refer to a collection or network of computing devices each comprising a processor or computational systems. The machine executable code or instructions may be executed by multiple computational systems or processors that may be within the same computing device or which may even be distributed across multiple computing devices.

Machine executable instructions or computer executable code may comprise instructions or a program which causes a processor or other computational system to perform an aspect of the present invention. Computer executable code for carrying out operations for aspects of the present invention may be written in any combination of one or more programming languages, including an object-oriented programming language such as Java, Smalltalk, C++ or the like and conventional procedural programming languages, such as the "C" programming language or similar programming languages and compiled into machine executable instructions. In some instances, the computer executable code may be in the form of a high-level language or in a pre-compiled form and be used in conjunction with an interpreter which generates the machine executable instructions on the fly. In other instances, the machine executable instructions or computer executable code may be in the form of programming for programmable logic gate arrays.

The computer executable code may execute entirely on the user's computer, partly on the user's computer, as a stand-alone software package, partly on the user's computer and partly on a remote computer or entirely on the remote computer or server. In the latter scenario, the remote computer may be connected to the user's computer through any type of network, including a local area network (LAN) or a wide area network (WAN), or the connection may be made to an external computer (for example, through the Internet using an Internet Service Provider).

Aspects of the present invention are described with reference to flowchart illustrations and/or block diagrams of methods, apparatus (systems) and computer program products according to embodiments of the invention. It is understood that each block or a portion of the blocks of the flowchart, illustrations, and/or block diagrams, can be implemented by computer program instructions in form of computer executable code when applicable. It is further under stood that, when not mutually exclusive, combinations of blocks in different flowcharts, illustrations, and/or block diagrams may be combined. These computer program instructions may be provided to a computational system of a general-purpose computer, special purpose computer, or other programmable data processing apparatus to produce a machine, such that the instructions, which execute via the computational system of the computer or other programmable data processing apparatus, create means for implementing the functions/acts specified in the flowchart and/or block diagram block or blocks.

These machine executable instructions or computer program instructions may also be stored in a computer readable medium that can direct a computer, other programmable data processing apparatus, or other devices to function in a particular manner, such that the instructions stored in the computer readable medium produce an article of manufacture including instructions which implement the function/act specified in the flowchart and/or block diagram block or blocks.

The machine executable instructions or computer program instructions may also be loaded onto a computer, other programmable data processing apparatus, or other devices to cause a series of operational steps to be performed on the computer, other programmable apparatus or other devices to produce a computer implemented process such that the instructions which execute on the computer or other programmable apparatus provide processes for implementing the functions/acts specified in the flowchart and/or block diagram block or blocks.
A 'user interface' as used herein is an interface which allows a user or operator to interact with a computer or computer system. A 'user interface' may also be referred to as a 'human interface device.' A user interface may provide information or data to the operator and/or receive information or data from the operator. A user interface may enable input from an operator to be received by the computer and may provide output to the user from the computer. In other words, the user interface may allow an operator to control or manipulate a computer and the interface may allow the computer to indicate the effects of the operator's control or manipulation. The display of data or information on a display or a graphical user interface is an example of providing information to an operator. The receiving of data through a keyboard, mouse, trackball, touchpad, pointing stick, graphics tablet, joystick, gamepad, webcam, headset, pedals, wired glove, remote control, and accelerometer are all examples of user interface components which enable the receiving of information or data from an operator.

A 'hardware interface' as used herein encompasses an interface which enables the computational system of a computer system to interact with and/or control an external computing device and/or apparatus. A hardware interface may allow a computational system to send control signals or instructions to an external computing device and/or apparatus. A hardware interface may also enable a computational system to exchange data with an external computing device and/or apparatus. Examples of a hardware interface include, but are not limited to: a universal serial bus, IEEE 1394 port, parallel port, IEEE 1284 port, serial port, RS-232 port, IEEE-488 port, Bluetooth connection, Wireless local area network connection, TCP/IP connection, Ethernet connection, control voltage interface, MIDI interface, analog input interface, and digital input interface.

A 'display' or 'display device' as used herein encompasses an output device or a user interface adapted for displaying images or data. A display may output visual, audio, and or tactile data. Examples of a display include, but are not limited to: a computer monitor, a television screen, a touch screen, tactile electronic display, Braille screen,
Cathode ray tube (CRT), Storage tube, Bi-stable display, Electronic paper, Vector display, Flat panel display, Vacuum fluorescent display (VF), Light-emitting diode (LED) displays, Electroluminescent display (ELD), Plasma display panels (PDP), Liquid crystal display (LCD), Organic light-emitting diode displays (OLED), a projector, and Head-mounted display.

K-space data is defined herein as being the recorded measurements of radio frequency signals emitted by atomic spins using the antenna of a Magnetic resonance apparatus during a magnetic resonance imaging scan. Magnetic resonance data is an example of tomographic medical image data.

A Magnetic Resonance Imaging (MRI) image or MR image is defined herein as being the reconstructed two- or three-dimensional visualization of anatomic data contained within the magnetic resonance imaging data. This visualization can be performed using a computer.

### BRIEF DESCRIPTION OF THE DRAWINGS

In the following preferred embodiments of the invention will be described, by way of example only, and with reference to the drawings in which:
Fig. 1 illustrates an example of a medical system;
Fig. 2 shows a flow chart which illustrates a method of using the medical system of Fig. 1;
Fig. 3 illustrates a further example of a medical system;
Fig. 4 shows a flow chart which illustrates a method of using the medical system of Fig. 3; and
Fig. 5 illustrates a further example of a medical system.

### DESCRIPTION OF EMBODIMENTS

Like numbered elements in these figures are either equivalent elements or perform the same function. Elements which have been discussed previously will not necessarily be discussed in later figures if the function is equivalent.

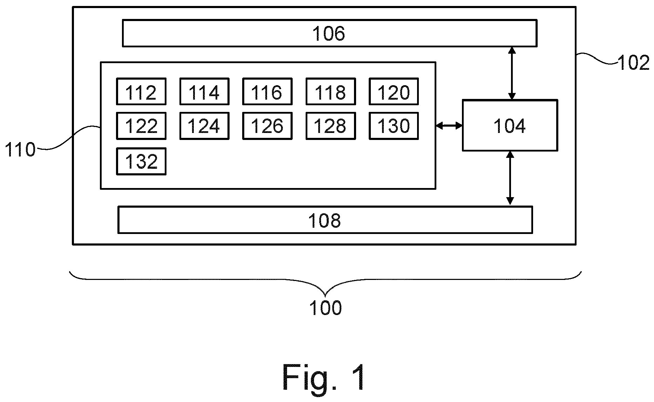

Fig. 1 illustrates an example of a medical system 100. The medical system 100 could for example be a cloud-based or virtual-based system. It could also be incorporated into a magnetic resonance imaging system. The medical system 100 is shown as comprising a computer 102. The computer 102 is intended to represent one or more computers located at one or more locations. The computer 102 is shown as comprising a computational system 104. The computational system 104 is intended to represent one or more computational systems or processing cores that may be located in one or more computer systems. The computational system 104 is shown as being in communication with a network interface 106 as well as an optional user interface 108 and a memory 110. The memory 110 is intended to represent various types of memory which may be accessible to the computational system 104.

The memory 110 is shown as containing machine-executable instructions 112. The memory 110 is further shown as containing a baseline anatomical measurement 114 and scan metadata 116 that are descriptive of a clinical magnetic resonance image. The memory 110 is further shown as containing an optional scan parameter configuration module 118. This could for example receive the scan metadata 116 and/or the baseline anatomical measurement 114 as input and then output scan parameters 120. The memory 110 is further shown as containing scan parameters 120. The memory 110 is further shown as containing subsequent k-space data 122 that was obtained from a low-field magnetic resonance imaging system. The memory 110 is further shown as containing a subsequent magnetic resonance image 124 that was reconstructed from the subsequent k-space data 122.

The memory 110 is further shown as containing a segmentation module 126 that was configured to receive the subsequent magnetic resonance image 124 as input and in response output a subsequent anatomical measurement 128. The memory 110 is shown as containing the subsequent anatomical measurement 128 that has been obtained by inputting the subsequent magnetic resonance image 124 into the segmentation module 126. The memory 110 is further shown as containing a predetermined amount 130 to be used as a threshold when comparing the subsequent anatomical measurement 128 to the baseline anatomical measurement 114. The memory 110 is further shown as containing a warning signal 132 that may be provided if the subsequent anatomical measurement 128 and the baseline anatomical measurement 114 vary by more than the predetermined amount 130. The warning signal 132 could for example be warnings or data that are presented to an operator. In another example the warning signal 132 may be commands which are then executed by a remote server or computer or even the low-field magnetic resonance imaging system.

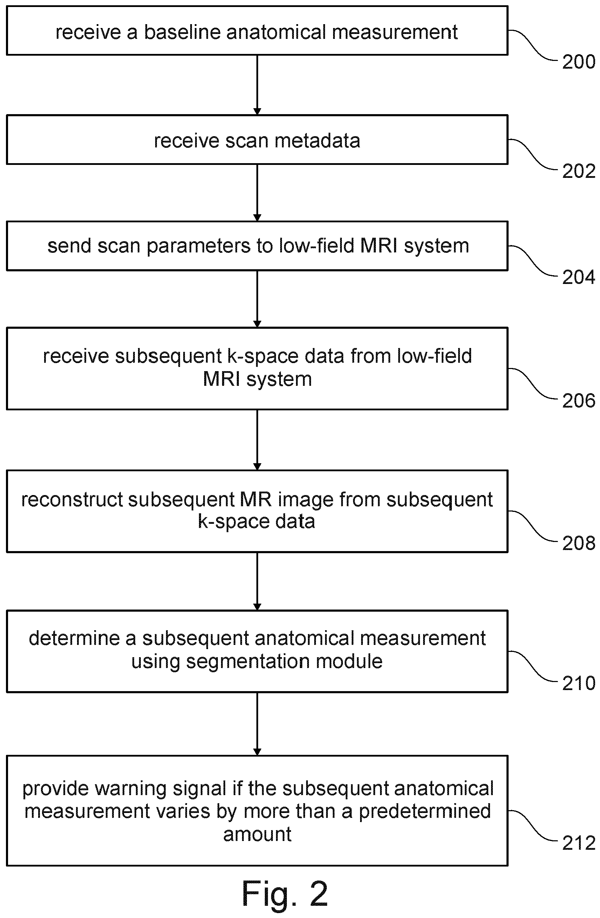

Fig. 2 shows a flowchart which illustrates a method of operating the medical system 100 of Fig. 1. First, in step 200, the baseline anatomical measurement 114 is received. The baseline anatomical measurement 114 is descriptive of a clinical magnetic resonance image of the subject. Next, in step 202, scan metadata 116, which is descriptive of the clinical magnetic resonance image, is received. Next, in step 204, the scan parameters 120 are sent via a network connection to a low-field magnetic resonance imaging system. Next, in step 206, the subsequent k-space data 122 is received from the low-field magnetic resonance imaging system via the network connection in response to sending it the scan parameters 120. Next, in step 208, the subsequent magnetic resonance image 124 is reconstructed from the subsequent k-space data 122. Then, in step 210, a subsequent anatomical measurement is determined in response to inputting the subsequent magnetic resonance image 124 into the segmentation module 126. Finally, in step 212, the warning signal 132 is provided if the subsequent anatomical measurement 128 varies from the baseline anatomical measurement 114 by more than the predetermined amount 130.

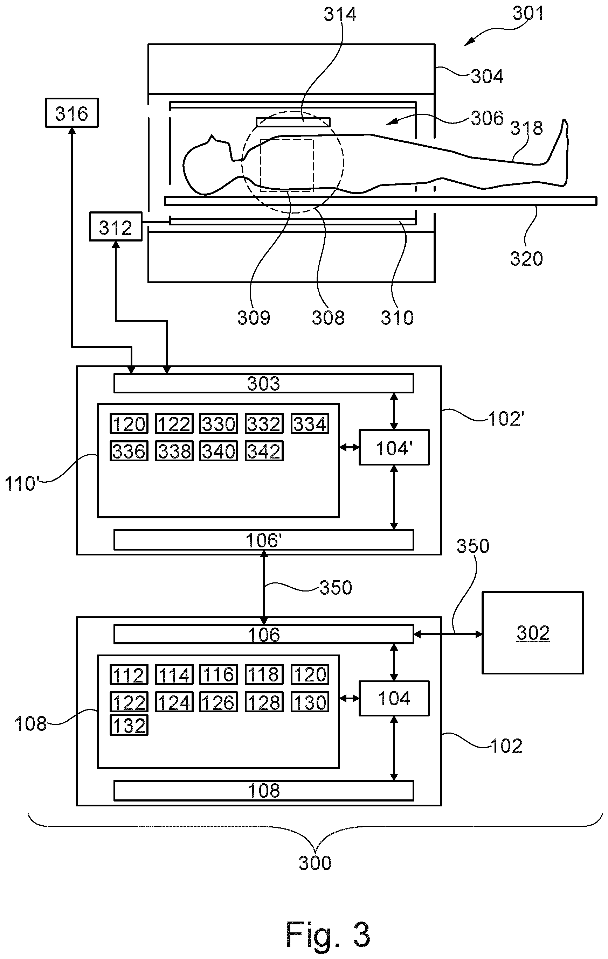

Fig. 3 illustrates a further example of the medical system 300. The medical system 300 is similar to that depicted in Fig. 1 except that it additionally comprises a low-field magnetic resonance imaging system 301 and an optional clinical magnetic resonance imaging system 302.

The low-field magnetic resonance imaging system 301 comprises a main magnet 304. The main magnet 304 is a superconducting cylindrical type magnet with a bore 306 through it. The use of different types of magnets is also possible; for instance it is also possible to use both a split cylindrical magnet and a so called open magnet. A split cylindrical magnet is similar to a standard cylindrical magnet, except that the cryostat has been split into two sections to allow access to the iso-plane of the magnet, such magnets may for instance be used in conjunction with charged particle beam therapy. An open magnet has two magnet sections, one above the other with a space in-between that is large enough to receive a subject: the arrangement of the two sections area similar to that of a Helmholtz coil. Open magnets are popular, because the subject is less confined. Inside the cryostat of the cylindrical magnet there is a collection of superconducting coils.

Within the bore 306 of the cylindrical magnet 304 there is an imaging zone 308 where the magnetic field is strong and uniform enough to perform magnetic resonance imaging. A field of view 309 is shown within the imaging zone 308. The k-space data that is acquired typically acquried for the field of view 309. The region of interest could be identical with the field of view 309 or it could be a sub volume of the field of view 309. A subject 318 is shown as being supported by a subject support 320 such that at least a portion of the subject 318 is within the imaging zone 308 and the field of view 309.

Within the bore 306 of the main magnet 304 there is also a set of magnetic field gradient coils 310 which is used for acquisition of preliminary k-space data to spatially encode magnetic spins within the imaging zone 308 of the magnet 304. The magnetic field gradient coils 310 connected to a magnetic field gradient coil power supply 312. The magnetic field gradient coils 310 are intended to be representative. Typically magnetic field gradient coils 310 contain three separate sets of coils for spatially encoding in three orthogonal spatial directions. A magnetic field gradient power supply supplies current to the magnetic field gradient coils. The current supplied to the magnetic field gradient coils 310 is controlled as a function of time and may be ramped or pulsed.

Adjacent to the imaging zone 308 is a radio-frequency coil 314 for manipulating the orientations of magnetic spins within the imaging zone 308 and for receiving radio transmissions from spins also within the imaging zone 308. The radio frequency antenna may contain multiple coil elements. The radio frequency antenna may also be referred to as a channel or antenna. The radio-frequency coil 314 is connected to a radio frequency transceiver 316. The radio-frequency coil 314 and radio frequency transceiver 316 may be replaced by separate transmit and receive coils and a separate transmitter and receiver. It is understood that the radio-frequency coil 314 and the radio frequency transceiver 316 are representative. The radio-frequency coil 314 is intended to also represent a dedicated transmit antenna and a dedicated receive antenna. Likewise the transceiver 316 may also represent a separate transmitter and receivers. The radio-frequency coil 314 may also have multiple receive/transmit elements and the radio frequency transceiver 316 may have multiple receive/transmit channels.

The low-field magnetic resonance imaging system 301 comprises the computer 102' that has a controller 104' that is connected to a hardware interface 303. The hardware interface 303 enables the controller 104' to send commands to the low-field magnetic resonance imaging system 300 and receive data in response. The controller 104' is shown as being further connected to a network interface 106' and a local memory 110'. The local memory 110' is intended to represent various types of memory that are accessible to the controller 104'.

The local memory 110' is shown as containing copies of the scan parameters 120 and the subsequent k-space data 122. The memory 110 is further shown as storing controller commands 330. The controller commands 330 are commands which enable the controller 104' to perform various tasks such as controlling the low-field magnetic resonance imaging system 300 and to perform data and image analysis. The local memory 110' is further shown as storing survey scan pulse sequence commands 332. Pulse sequence commands are commands which enable the controller 104' to control the low-field magnetic resonance imaging system 300 to acquire k-space data. The survey scan pulse sequence commands 332 are therefore commands which enable the low-field magnetic resonance imaging system 300 to perform a survey scan.

The local memory 110' is further shown as containing survey scan k-space data 334 that was acquired by controlling the magnetic resonance imaging system 300 with survey scan pulse sequence commands 332. The memory 110' is further shown as containing a survey scan image 336 that is a magnetic resonance image reconstructed from the survey scan k-space data 334. The memory 110' is further shown as containing a location of predetermined anatomical landmarks 338 in the survey scan image 336. Various segmentation and landmark recognition techniques may be used to identify this location 338 in the survey scan image 336. For example, a template image may be used as well as various segmentation algorithms. The memory 110' is further shown as containing acquisition pulse sequence commands 340. The acquisition pulse sequence commands 340 may be used to control the low-field magnetic resonance imaging system 300 to acquire the subsequent k-space data 122. The memory 110' is further shown as containing modified acquisition pulse sequence commands 342 that have been modified with the scan parameters 120. This for example may be used for precise location of the field of view 309 during the acquisition of the k-space data.

The low-field magnetic resonance imaging system 300, the computer 102 and the optional clinical magnetic resonance imaging system 302 are shown as being connected via a network connection 350. The baseline anatomical measurement 114 and the scan metadata 116 may have been received from the clinical magnetic resonance imaging system 302 via the network connection 350.

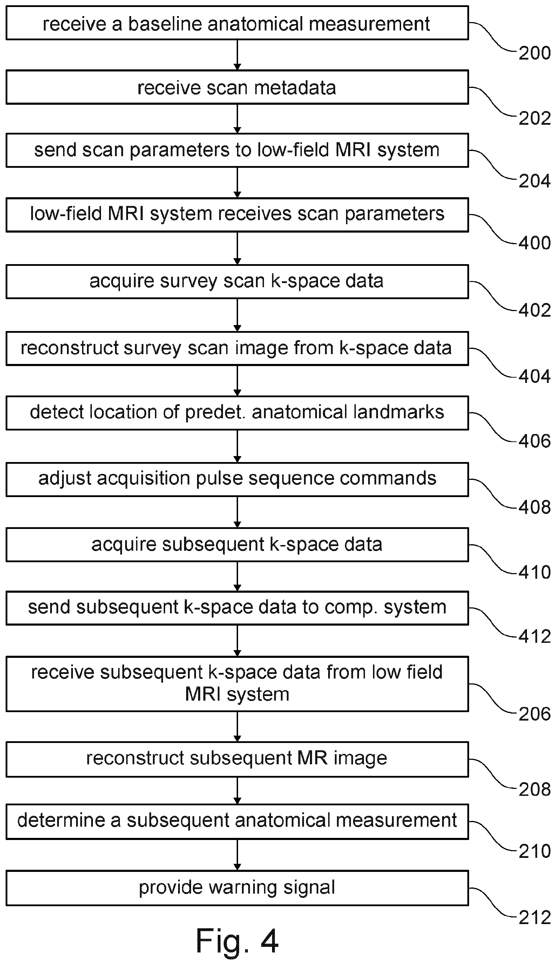

Fig. 4 shows a flowchart which illustrates a method of operating the medical system 300 of Fig. 3. The method illustrated in Fig. 4 is similar to that illustrated in Fig. 2 with additional steps that are performed. Initially, steps 200, 202, and 204 are performed as was illustrated in Fig. 2. After step 204 the method proceeds to step 400. In step 400 the scan parameters 120 are received by the low-field magnetic resonance imaging system via the network connection 350. Then, in step 402, the survey scan k-space data 334 is acquired by controlling the low-resolution magnetic resonance imaging system 300 with the survey scan pulse sequence commands 332. Next, in step 404, the survey scan image 336 is reconstructed from the survey scan k-space data 334. Next, in step 406, the location 338 of the predetermined anatomical landmarks is detected in the survey scan image 336. Then, in step 408, the acquisition pulse sequence commands 340 are adjusted using the location of the predetermined anatomical landmarks 338, and/or the scan parameters 120.

This may include such things as the second resolution, and the scan coordinates referenced to the predetermined anatomical landmarks of the subject. Next, in step 410, the subsequent k-space data 122 is acquired by controlling the low-resolution magnetic resonance imaging system with the acquisition pulse sequence commands that have been modified 342. Next, in step 412, the subsequent k-space data 122 is sent from the low-field magnetic resonance imaging system 300 via the network connection 350 to the computational system 104. After step 412 is performed, steps 206, 208, 210, and 212 are performed as is illustrated in Fig. 2.

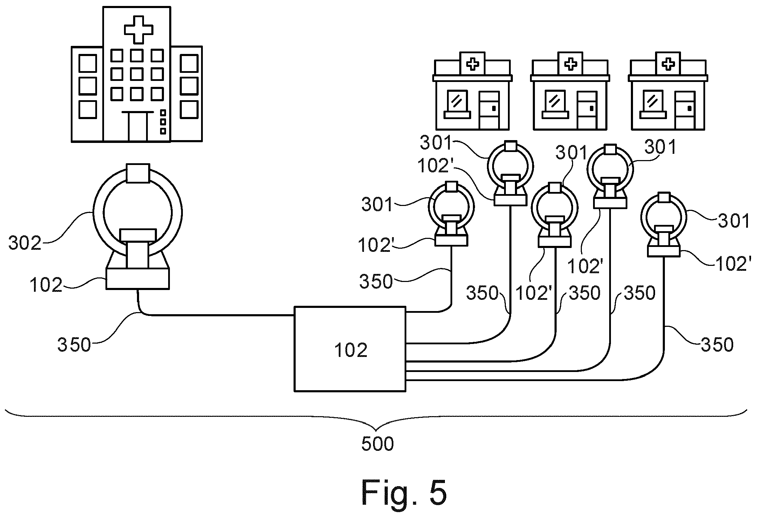

Fig. 5 illustrates a further example of a medical system 500 where the computer 102 functions as a cloud-based system that is connected to a clinical magnetic resonance imaging system 302 as well as multiple of the low-field magnetic resonance imaging system 300. Each low-field magnetic resonance imaging system 300 has a controller 104' that is connected to the computer 102 via the network connection 350. The system illustrated in Fig. 5 may have the advantage that the clinical magnetic resonance imaging system 302 is not needed to repeatedly acquire the baseline anatomical measurement 114. A subject could go into any one of the low-field magnetic resonance imaging systems 300 and have the subsequent anatomical measurement 128 measured.

While the invention has been illustrated and described in detail in the drawings and foregoing description, such illustration and description are to be considered illustrative or exemplary and not restrictive; the invention is not limited to the disclosed embodiments.

Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. A single processor or other unit may fulfill the functions of several items recited in the claims. The mere fact that certain measures are recited in mutually different dependent claims does not indicate that a combination of these measured cannot be used to advantage. A computer program may be stored/distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

### REFERENCE SIGNS LIST

- 100: medical system
- 102: computer
- 104: computational system
- 104': controller
- 106: network interface
- 108: user interface
- 110: memory
- 110': local memory
- 112: machine executable instructions
- 114: baseline anatomical measurement
- 116: scan metadata
- 118: scan parameter configuration module
- 120: scan parameters
- 122: subsequent k-space data
- 124: subsequent magnetic resonance image
- 126: segmentation module
- 128: subsequent anatomical measurement
- 130: predetermined amount
- 132: warning signal
- 200: receive a baseline anatomical measurement descriptive of a clinical magnetic resonance image of a subject
- 202: receive scan metadata descriptive of the clinical magnetic resonance image of the subject
- 204: send scan parameters via a network connection to a low-field magnetic resonance imaging system
- 206: receive subsequent k-space data from the low-field magnetic resonance imaging system via the network connection in response to sending the scan parameters
- 208: reconstruct a subsequent magnetic resonance image from the subsequent k-space data
- 210: determine a subsequent anatomical measurement in response to inputting the subsequent magnetic resonance image into the segmentation module
- 212: provide a warning signal if the subsequent anatomical measurement varies from the baseline anatomical measurement by more than a predetermined amount
- 300: medical system
- 301: low-field magnetic resonance imaging system
- 302: clinical magnetic resonance imaging system
- 303: hardware interface
- 304: main magnet
- 306: bore of magnet
- 308: imaging zone
- 309: field of view
- 310: magnetic field gradient coils
- 312: magnetic field gradient coil power supply
- 314: radio-frequency coil
- 316: transceiver
- 318: subject
- 320: subject support
- 330: controller commands
- 332: survey scan pulse sequence commands
- 334: survey scan k-space data
- 336: survey scan image
- 338: location of predetermined anatomical landmarks
- 340: measurement pulse sequence commands
- 342: modified measurement pulse sequence commands
- 400: receive the scan parameters via the network connection
- 402: acquire survey scan k-space data by controlling the low-resolution magnetic resonance imaging system with the survey scan pulse sequence commands
- 404: reconstruct a survey scan image from the survey scan k-space data
- 406: detect a location of the predetermined anatomical landmarks of the subject in the survey scan image
- 408: adjust the acquisition pulse sequence commands using the location of the predetermined anatomical landmarks of the subject in the survey scan image, the second resolution, and the scan coordinates referenced to predetermined anatomical landmarks of the subject
- 410: acquire the subsequent k-space data by controlling the low-resolution magnetic resonance imaging system with the acquisition pulse sequence commands
- 412: send the subsequent k-space data to the computational system via the network connection
- 350: network connection
- 500: medical system

## Claims

1. A medical system (100, 300, 500) comprising:
- a memory (110) storing machine executable instructions (112);
- a computational system (104), wherein execution of the machine executable instructions causes the computational system to:
- receive (200) a baseline anatomical measurement (114) descriptive of a clinical magnetic resonance image of a subject (318);
- receive (202) scan metadata (116) descriptive of the clinical magnetic resonance image of the subject, wherein the scan metadata comprises scan coordinates referenced to predetermined anatomical landmarks of the subject, wherein the scan metadata is descriptive of the clinical magnetic resonance image having a first resolution and a first signal to noise ratio;
- send (204) scan parameters via a network connection (350) to a low-field magnetic resonance imaging system (301), wherein the scan parameters comprise a second resolution and a second signal to noise ratio, wherein the first resolution is higher than the second resolution and/or the first signal to noise ratio is higher than the second signal to noise ratio;
- receive (206) subsequent k-space data (122) from the low-field magnetic resonance imaging system via the network connection in response to sending the scan parameters;
- reconstruct (208) a subsequent magnetic resonance image (124) from the subsequent k-space data;
- determine (210) a subsequent anatomical measurement (128) in response to inputting the subsequent magnetic resonance image into the segmentation module; and
- provide (212) a warning signal (132) if the subsequent anatomical measurement varies from the baseline anatomical measurement by more than a predetermined amount.

2. The medical system of claim 1, wherein the medical system further comprises the low-resolution magnetic resonance imaging system, wherein the low-resolution magnetic resonance imaging system comprises a local memory (110') and a controller (104'); wherein the local memory stores survey scan pulse sequence commands (332) and measurement pulse sequence commands (340), wherein the local memory further stores controller commands (330), wherein execution of the controller commands causes the controller to:
- receive (400) the scan parameters via the network connection;
- acquire (402) survey scan k-space data by controlling the low-resolution magnetic resonance imaging system with the survey scan pulse sequence commands;
- reconstruct (404) a survey scan image (336) from the survey scan k-space data;
- detect (406) a location (338) of the predetermined anatomical landmarks of the subject in the survey scan image;
- adjust (408) the acquisition pulse sequence commands using the location of the predetermined anatomical landmarks of the subject in the survey scan image, the second resolution, and the scan coordinates referenced to predetermined anatomical landmarks of the subject;
- acquire (410) the subsequent k-space data by controlling the low-resolution magnetic resonance imaging system with the modified acquisition pulse sequence commands;
- send (412) the subsequent k-space data to the computational system via the network connection.

3. The medical system of claim 2, wherein the low-resolution magnetic resonance imaging system comprises a main magnet (304) configured for generating a main magnetic field, wherein the main magnetic field has a strength of 0.6 Tesla or less, and wherein the wherein the main magnetic field preferably has a strength of 0.2 Tesla or less.

4. The medical system of claim 2 or 3, wherein the controller is configured to: perform the acquisition of the subsequent k-space data, and send the subsequent k-space data to the computational system automatically.

5. The medical system of any one of the preceding claims, wherein the memory further stores a scan parameter configuration module (118); wherein the scan parameter configuration module is configured to output the scan parameters in response to receiving the second resolution, the scan coordinates, and the baseline anatomical measurement; wherein execution of the machine executable instructions further causes the computational system to receive the scan parameters in response to inputting the second resolution, the scan coordinates, and the baseline anatomical measurement into the scan parameter configuration module.

6. The medical system of claim 5, wherein the scan parameter configuration module is at least partially implemented as a lookup table, a neural network, or an expert system.

7. The medical system of claim 5 or 6, wherein the scan parameters comprise a selection of a pulse sequence type.

8. The medical system of any one of the preceding claims, wherein the computational system is implemented as a cloud computing system.

9. The medical system of any one of the preceding claims, wherein the segmentation module is implemented as a neural network.

10. The medical system of claim 9, wherein neural network is trained by repeatedly:
- receiving a training clinical magnetic resonance image or training clinical k-space data, wherein the training clinical magnetic resonance image has the first resolution;
- receiving a training anatomical measurement descriptive of the baseline anatomical measurement on the training clinical magnetic resonance image;
- calculate a simulated subsequent magnetic resonance image with the second resolution from either the training clinical magnetic resonance image or the training clinical k-space data;
- construct training data from pairs of the simulated subsequent magnetic resonance image and the training anatomical measurement;
- train the neural network using the training data.

11. The medical system of any one of the preceding claims wherein the medical system further comprises a clinical magnetic resonance imaging system, wherein the clinical magnetic resonance imaging system is configured to acquire the clinical magnetic resonance image of the subject and determine the baseline anatomical measurement from the clinical magnetic resonance image, wherein the clinical magnetic resonance image is configure to provide the baseline anatomical measurement to the computational system.

12. A method of medical imaging, wherein the method comprises:
- receiving (200) a baseline anatomical measurement (114) descriptive of a clinical magnetic resonance image of a subject (318);
- receiving (202) scan metadata (116) descriptive of the clinical magnetic resonance image of the subject, wherein the scan metadata comprises scan coordinates referenced to predetermined anatomical landmarks of the subject, wherein the scan metadata is descriptive of the clinical magnetic resonance image having a first resolution;
- sending (204) scan parameters to a low-field magnetic resonance imaging system ( 301) via a network connection (350), wherein the scan parameters comprise a second resolution and the scan coordinates reference to the predetermined anatomical landmarks of the subject, wherein the first resolution is higher than the second resolution;
- receiving (206) subsequent k-space data (122) from the low-field magnetic resonance imaging system via the network connection in response to sending the scan parameters;
- reconstructing (208) a subsequent magnetic resonance image (124) from the subsequent k-space data;
- determining (210) a subsequent anatomical measurement (128) in response to inputting the subsequent magnetic resonance image into the segmentation module; and
- providing (212) a warning signal (132) if the subsequent anatomical measurement varies from the baseline anatomical measurement by more than a predetermined amount.

13. The method of claim 12, wherein the medical system further comprises the low-resolution magnetic resonance imaging system, wherein the method further comprises:
- receiving (400) the scan parameters via the network connection;
- acquiring (402) survey scan k-space data by controlling the low-resolution magnetic resonance imaging system with survey scan pulse sequence commands;
- reconstructing (404) a survey scan image (336) from the survey scan k-space data;
- detecting (406) a location (338) of the predetermined anatomical landmarks of the subject in the survey scan image;
- adjusting (408) the acquisition pulse sequence commands using the location of the predetermined anatomical landmarks of the subject in the survey scan image, the second resolution, and the scan coordinates referenced to the predetermined anatomical landmarks of the subject;
- acquiring (410) the subsequent k-space data by controlling the low-resolution magnetic resonance imaging system with the acquisition pulse sequence commands; and
- sending (412) the subsequent k-space data to the computational system via the network connection.

14. A computer program product comprising machine executable instructions for execution by a computational system controlling a medical system (100, 300, 500), wherein execution of the machine executable instructions causes the computational system to:
- receive (200) a baseline anatomical measurement (114) descriptive of a clinical magnetic resonance image of a subject (318);
- receive (202) scan metadata (116) descriptive of the clinical magnetic resonance image of the subject, wherein the scan metadata comprises scan coordinates referenced to predetermined anatomical landmarks of the subject, wherein the scan metadata is descriptive of the clinical magnetic resonance image having a first resolution;
- send (204) scan parameters to a low-field magnetic resonance imaging system (301) via a network connection (350), wherein the scan parameters comprise a second resolution and the scan coordinates reference to the predetermined anatomical landmarks of the subject, wherein the first resolution is higher than the second resolution;
- receive (206) subsequent k-space data (122) from the low-field magnetic resonance imaging system via the network connection in response to sending the scan parameters;
- reconstruct (208) a subsequent magnetic resonance image (124) from the subsequent k-space data;
- determine (210) a subsequent anatomical measurement (128) in response to inputting the subsequent magnetic resonance image into the segmentation module; and
- provide (212) a warning signal (132) if the subsequent anatomical measurement varies from the baseline anatomical measurement by more than a predetermined amount.
